# EUROPEAN PATENT SPECIFICATION

(11) **EP 3 717 659 B1**
(45) Date of publication and mention of the grant of the patent: **03.08.2022**
(21) Application number: 18819276.9
(22) Date of filing: 30.11.2018
(51) Int. Cl.: C12Q 1/18, C12Q 1/04

(54) **A METHOD FOR DETERMINING MICROBIAL SUSCEPTIBILITY TO ANTIBIOTIC AGENTS**
VERFAHREN ZUR BESTIMMUNG DER MIKROBIELLEN EMPFINDLICHKEIT GEGENÜBER ANTIBIOTISCHEN MITTELN
PROCÉDÉ DE DÉTERMINATION DE LA SENSIBILITÉ DE MICROBES À DES AGENTS ANTIBIOTIQUES

(30) Priority: 30.11.2017 SE 1751474
(43) Date of publication of application: 07.10.2020
(73) Proprietor: Ascilion AB, 164 25 Kista (SE)
(72) Inventor: RANGSTEN, Pelle, 743 40 Storvreta (SE); RENLUND, Markus, 184 60 Åkersberga (SE); FRANZÉN, Mikael, 178 51 Ekerö (SE); HILLMERING, Mikael, 192 73 Sollentuna (SE)
(74) Representative: Zacco Sweden AB
(86) International application number: PCT/SE2018/051237
(87) International publication number: WO 2019/108125

(56) References cited:
- EP-A1- 0 558 827
- EP-A1- 2 821 499
- EP-A1- 3 070 175
- WO-A1-90/03441
- WO-A1-2005/049809
- JP-A- H07 222 599
- US-A- 4 132 599
- ROSARIO ESPOSITO ET AL: "Glucose Sensing by Time-Resolved Fluorescence of Sol-Gel Immobilized Glucose Oxidase", SENSORS, vol. 11, no. 4, 24 March 2011 (2011-03-24) , pages 3483-3497, XP055547722, DOI: 10.3390/s110403483
- Dorey Lucy ET AL: "Impact of growth matrix on pharmacodynamics of antimicrobial drugs for pig pneumonia pathogens", BMC Veterinary Research, vol. 13, no. 1, 23 June 2017 (2017-06-23), XP055818207, DOI: 10.1186/s12917-017-1086-4 Retrieved from the Internet: URL:https://www.ncbi.nlm.nih.gov/pmc/artic les/PMC5481914/pdf/12917_2017_Article_1086 .pdf>

## Description

### TECHNICAL FIELD

The present invention generally relates to the field of microbial measurements, and more specifically to the field of determining microbial susceptibility to antibiotic agents.

### BACKGROUND ART

A common problem in clinical settings is to measure the efficiency of an antibiotic on a sample with bacteria. This is conventionally performed by providing a sample with bacteria to an Agar plate with different disks comprising antibiotic agents. The agar plate is then placed in an environment suitable for microbial growth, after an incubation time of approximately 24 hours the agar plate is then analysed. If the antibiotic is efficient a ring of dead bacteria is formed around each disk with antibiotic agent. The diameter of the ring is proportional to the efficiency of the antibiotic.

It is known in the art (e.g. JP57068796, WO2016/150871) to use pH indicators to detect the acidic degradation products of glucose metabolism in *Enterobacteriaceae* when cultured in the presence or absence of antibiotics, in order to assess antibiotic susceptibility.

Sine et al. have suggested prediction of antibiotic resistance through measurement of glucose consumption (Sine et al., poster 099, 111^{th} General Meeting American Society for Microbiology, 2011).

WO2015/107054 discloses a mass spectrometric method for determining microbial resistance to antibiotics. The method is based on growing microbes in a culture medium and mass spectrometric measurement of a nutrient component, such as a peptide, or a chemically modified variant of the nutrient component, wherein a decrease in the nutrient component or an increase in the chemically modified variant of the nutrient component indicates resistance to the antibiotic. This method requires removal of culture medium for measurement in a separate mass spectrometer. The method also requires a fully synthetic culture medium, which has clean mass spectra without much chemical background noise.

Esposito et al., Sensors, 2011, 11, pp. 3483-3497 describe a monolithic silica gel matrix with entrapped glucose oxidase (GOD) constructed as a bioactive element in an optical biosensor for glucose determination.

Dorey and Lees, BMC Veterinary Research (2017) 13:192 compares Minimum Inhibitory Concentration (MIC) values in several broths of differing chemical composition for the pig pathogens *A. pleuropneumoniae* and *P. multocida* and the drugs florfenicol, oxytetracycline, and marbofloxacin.

### SUMMARY OF THE INVENTION

There exists a need for methods for rapid assessments of whether a microorganism is susceptible to an antibiotic. Such methods preferably provide the user with information on what type of antibiotic may be used and the concentrations that are needed to inhibit the growth of, or kill, the microorganism.

The present invention aims to provide such methods.

Accordingly, the present invention relates to a method for analysing susceptibility of a glucose metabolizing microorganism to at least one antibiotic agent, comprising cultivating the microorganism in a culture medium in the presence and absence of antibiotic agent and measuring the rate of change in glucose concentration in the respective media. The invention further relates to a method for assessing efficacy of a candidate antibiotic agent in treatment of a medical condition caused or complicated by an infection by using the method according to the invention, as well as a culture medium suitable for use in the methods.

In a first aspect, the present invention thus relates to a method for analysing susceptibility of a glucose metabolizing microorganism to at least one antibiotic agent, comprising
a) Cultivating said glucose metabolizing microorganism in a reference culture medium, comprising glucose and not comprising the antibiotic agent; and measuring the glucose concentration in the reference culture medium to obtain time-resolved glucose concentration data, wherein said cultivation extends for a period of time sufficient to observe a decrease in glucose concentration in the reference culture medium;
b) Simultaneously cultivating said glucose metabolizing microorganism in a culture medium contained in a culture medium container, in the presence of a predetermined concentration of an antibiotic agent and in the presence of glucose;
c) Measuring the concentration of glucose in the culture medium in said culture medium container at least when the glucose concentration in the reference culture medium has decreased below a predetermined threshold; and
d) calculating a susceptibility score reflecting the susceptibility of the glucose metabolizing microorganism to the antibiotic agent based on a relation between the predetermined concentration of the agent with antibiotic activity, the glucose concentration data obtained in step c), and the time-resolved glucose concentration data obtained in step a).

If the microorganisms are susceptible to the antibiotic activity of the agent, the glucose concentration will be constant or decrease only slowly over time as no or little glucose metabolism occurs. If the microorganisms are resistant to the antibiotic activity of the agent then the glucose concentration will decrease significantly over time due to the glucose consumption by the microorganisms in the sample.

In one embodiment, step c) comprises obtaining time-resolved glucose concentration data for the culture medium in the culture medium container during at least a part of the time period during which the glucose metabolizing microorganism is cultured.

In one embodiment, the method further comprises obtaining time-resolved glucose concentration reference data for culture medium in a reference container comprising no glucose metabolizing microorganism.

In one embodiment, said glucose metabolizing microorganism is cultured in a plurality of culture medium containers and wherein said antibiotic agent is present in each culture medium container in an amount that is predetermined for each culture medium container.

In one embodiment, the plurality of culture medium containers together contain a series of concentrations of the antibiotic agent.

In one embodiment, said glucose metabolizing microorganism is cultivated in a plurality of culture medium containers and wherein the culture medium containers together contain a plurality of different antibiotic agents.

In one embodiment, the susceptibility score is a qualitative susceptibility score. In one embodiment, the qualitative susceptibility score is defined for a specific concentration of a specific agent with antibiotic activity.

In one embodiment, the susceptibility score is a quantitative susceptibility score, such as a minimum inhibitory concentration (MIC), or a minimum lethal concentration (MLC).

In one embodiment, the susceptibility score is calculated based on time-resolved glucose concentration data from a plurality of culture medium containers comprising the microorganism and together comprising a serial dilution of the antibiotic agent, a reference culture medium container comprising the microorganism and not comprising the antibiotic agent, and a blank culture medium container not comprising the microorganism and not comprising the antibiotic agent.

In one embodiment, , the measurement of glucose concentration involves a frequency measurement indicative of the glucose concentration. In one embodiment, the frequency measurement is a measurement of a resonance frequency of the culture medium container. In one embodiment, the frequency measurement is an electrical high frequency measurement. In one embodiment, the measurement of glucose concentration is performed with an enzyme electrode.

In one embodiment, the glucose concentration is measured within the culture medium container.

In one embodiment, the culture medium and reference culture medium is Mueller-Hinton broth or Brain Heart Infusion, supplemented with glucose.

In a further aspect, the invention relates to a method for assessing efficacy of a candidate antibiotic agent in treatment of a medical condition caused or complicated by an infection, comprising providing a body fluid sample from a subject known or suspected of suffering from an infection and obtaining a susceptibility score reflecting the susceptibility of any glucose metabolizing microorganism present in said sample to said candidate antibiotic agent using a method according to the above, wherein the susceptibility score indicates the efficacy of the candidate antibiotic agent in the treatment of the medical condition.

In one embodiment, the medical condition is selected from the group consisting of septicaemia, pneumonia, endocarditis, peritonitis, and meningitis.

In a further aspect, the invention relates to the use of a liquid culture medium comprising, or consisting of: Brain Heart Infusion broth with a glucose concentration of 1 mg glucose per mL culture medium; or Mueller-Hinton broth with a glucose concentration of 0.5-2.0 mg glucose per mL culture medium, such as 1 mg/mL, in the methods according to the invention.

Preferred embodiments are as set out in the dependent claims.

### BRIEF DESCRIPTION OF THE DRAWINGS

Embodiments of the invention will now be explained in detail, by way of example only, with reference to the accompanying figures, in which:
Figure 1 is a flow diagram illustrating a method according to the invention.
Figure 2 is a flow diagram illustrating a method according to an embodiment of the invention.
Figure 3 is a flow diagram illustrating a method according to an embodiment of the invention.
Figure 4 is a diagram showing time-resolved glucose concentration data obtained for *E.coli*
Figure 5 is a diagram showing time-resolved glucose concentration data obtained for *E.faecalis*
Figure 6 is a diagram showing time-resolved glucose concentration data obtained for *S.aureus.*

### DEFINITIONS

All terms used herein are intended to have the meaning given to them by the person skilled in the art. For the sake of clarity, a few terms are further defined below.

The terms "antibiotic agent" or "agent with antibiotic activity" shall be considered equivalent and relate to agents that suppress or inibit the growth or viability of microorganisms, such as bacteria, archaebacteria, protozoa and yeasts. Such agents include chemical compounds, ultraviolet light, radiation, heating, microwaves etc.

### DETAILED DESCRIPTION

The present invention provides rapid methods for analysing susceptibility of a glucose metabolizing microorganism to an antibiotic agent or agents. Such methods are useful in a number of settings, i.a. analysis of patient samples and environmental samples.

One important application of the method according to the invention is in connection with treatment of human and animal patients suffering from serious and sometimes life threatening infection such as sepsis. For these patients it is imperative that adequate antibiotic therapy can be initiated as soon as possible, and consequently methods for rapid assessment of what antibiotic to use is needed. The present invention aims to provide and facilitate such methods.

Another application of the method according to the invention is in analysis of environmental samples. The presence of microorganisms that are resistant to antibiotics is a problem in a number of environments, such as hospitals, animal stables, etc. The present invention also aims to provide methods that can be used to analyse such samples.

The present invention thus in a first aspect relates to method for analysing susceptibility of a glucose metabolizing microorganism to at least one antibiotic agent, comprising
a) Cultivating said glucose metabolizing microorganism in a reference culture medium, comprising glucose and not comprising the antibiotic agent, for a period of time sufficient to observe a decrease in glucose concentration in the reference culture medium;
b) Simultaneously cultivating said glucose metabolizing microorganism in a culture medium contained in a culture medium container, in the presence of a predetermined concentration of an antibiotic agent and in the presence of glucose;
c) Measuring the concentration of glucose in the culture medium in said culture medium container at least when the glucose concentration in the reference medium has decreased below a predetermined threshold; and
d) Calculating a susceptibility score reflecting the susceptibility of the glucose metabolizing microorganism to the antibiotic agent based on a relation between the predetermined initial glucose concentration and the measured glucose concentration.

This provides the advantage that the microbial growth and viability may be determined much faster compared to known methods in the art.

In one embodiment, step a) comprises obtaining time-resolved glucose concentration reference data for the culture medium in a first reference container comprising the glucose metabolizing microorganism.

In one embodiment, the glucose concentration measurement of step c) is performed at least when the glucose concentration in the first reference container has decreased below a predetermined threshold. This threshold could be set at a fraction of the initial glucose concentration, such as 90%, 80%, 75%, or 50%. The threshold may also be set as an absolute glucose concentration determined based on the specific conditions of the implementation of the method

In one embodiment, step c) above comprises obtaining time-resolved glucose concentration data for the culture medium in the culture medium container during at least a part of the time period during which the glucose metabolizing microorganism is cultured.

Time-resolved glucose concentration data in the culture medium and reference culture medium is typically obtained by measuring the glucose concentration continuously or at regular time intervals, such as every minute, every 5 minutes, every 10 minutes, every 15 minutes, every 30 minutes, or every 60 minutes. The timing of measuring glucose concentration may also be made dependent on e.g. the glucose concentration in the reference culture medium is observed to be in a certain range, then glucose concentrations are measured in the remaining culture media as well.

In one embodiment, the method comprises obtaining time-resolved glucose concentration reference data for culture medium in a reference container comprising no glucose metabolizing microorganism. This is done in order to have a blank reference that can be used as comparison.

In one embodiment of the invention, the glucose metabolizing microorganism is cultured in a plurality of culture medium containers and the antibiotic agent is present in each culture medium container in an amount that is predetermined for each culture medium container. Preferably, the plurality of culture medium containers together contain a series of concentrations of the antibiotic agent. This series of concentrations may be a serial dilution where each concentration is a twofold dilution of a higher concentration.

In one embodiment, the method comprises cultivating the glucose metabolizing microorganism in a plurality of culture medium containers and wherein the culture medium containers together contain a plurality of different antibiotic agents. This allows for assessing susceptibility scores for the microorganism in relation to a range of antibiotics in a single run of the method.

In one embodiment, the susceptibility score is a qualitative susceptibility score. A qualitative susceptibility score can be binary, i.e. classifying the microorganism as either susceptible or non-susceptible/resistant to the antibiotic activity of an agent at a certain concentration. It can also use further classifications, such as "intermediate susceptibility".

In one embodiment, the susceptibility score is a quantitative susceptibility score. A quantitative susceptibility score is a numerical value reflecting the degree of susceptibility to the antibiotic agent. Two typical susceptibility scores that can be used are the Minimum Inhibitory Concentration (MIC) which is the lowest concentration of the antibiotic that inhibits its growth, and the Minimum Lethal Concentration (MLC) or Minimum Bactericidal Concentration (MBC) in case of bacteria, which corresponds to the lowest concentration of the antibiotic that kills the microorganism. MIC and MLC/MBC values are commonly determined in Antibiotic Susceptibility Test procedures and used in clinical practice to determine the necessary dose needed to treat an infection (see e.g. Finberg and Guharoy, Clinical use of Anti-infective Agents, 1^{st} ed. 2012, SpringerNature, Germany).

In one embodiment, the quantitative susceptibility score is calculated based on time-resolved glucose concentration data from a plurality of culture medium containers comprising the microorganism and together comprising a serial dilution of the antibiotic agent, a reference culture medium container comprising the microorganism and not comprising the antibiotic agent, and a blank culture medium container not comprising the microorganism and not comprising the antibiotic agent.

In one embodiment, the measurement of glucose concentration involves a frequency measurement indicative of the glucose concentration. The frequency measurement may be a measurement of a resonance frequency of the culture medium container, and/or an electrical high frequency measurement. How to perform such measurements are further described below.

In one embodiment, measurement of glucose concentration is performed with an enzyme electrode. How to perform such measurements are further described below.

In one embodiment, the glucose concentration is measured within the culture medium container. This obviates the need for transfer of culture medium to a separate measurement or detection device.

In one embodiment, the culture medium and reference culture medium is Mueller-Hinton broth or Brain Heart Infusion broth, supplemented with glucose. These media can be supplemented with glucose to a final glucose concentration of 0.5-2.0 mg/mL, preferably 1.0 mg/mL. Brain Heart Infusion (BHI) is a general-purpose liquid medium used in the cultivation of fastidious and non-fastidious microorganisms, including aerobic and anaerobic bacteria, from a variety of clinical and nonclinical materials. Mueller-Hinton broth (MHB) is a standard medium used in Antibiotic Susceptibility Testing. Both media are well-known in the art and described i.a. in Handbook of Microbiological Media by Ronald M. Atlas, 3rd ed, 2004 CRC Press, Boca Raton, FL, USA.

In one aspect, the invention relates to a method for assessing efficacy of a candidate antibiotic agent in treatment of an infection, comprising providing a body fluid sample from a subject known or suspected of suffering from an infection and obtaining a susceptibility score reflecting the susceptibility of any glucose metabolizing microorganism present in said sample to said candidate antibiotic agent using a method according to the above, wherein the susceptibility score indicates the efficacy of the candidate antibiotic agent in the treatment of the infection.

In a presently preferred embodiment of this aspect, the invention relates to a method for assessing efficacy of a candidate antibiotic agent in treatment of sepsis and the susceptibility score indicates the efficacy of the candidate antibiotic agent in the treatment of sepsis.

Variuos embodiments of the invention and its parts will now be described for illustrative purposes.

Initially, a sample comprising, or suspected of comprising, a microorganism is provided or obtained. The sample could be any sample comprising, or suspected of comprising, a microorganism such as body fluids, including but not limited to whole blood, serum, plasma, cerebrospinal fluid, lymph, sweat, tears, faeces and urine; and environmental samples, including but not limited to soil, water and swabs. In a preferred embodiment the sample is a whole blood sample.

Depending on the origin and type of sample, it may be desirable to perform an initial cultivation in order to increase the cell count of the microorganisms in the sample or obtain a subsample of a single Colony Forming Unit (cfu). For instance, the sample could be divided into aliquots, serially diluted and plated onto Petri dishes containing suitable solid culture medium and cultivated over night to yield distinguishable colonies stemming from a single cfu, as known in the art. Samples from single colonies may then be transferred to a new culture medium using a properly sterilized loop to obtain a new culture originating from a single cfu. The sample may also be transferred to a blood culture medium for initial cultivation. Such cultivation is *per se* well-known in the art and is usually performed in so-called blood culture bottles (or "BC bottles"). Blood culture media include tryptic soy broth and thioglycollate broth, and are commercially available from a number of providers. A sample from this culture may then be used as a starting material in the method according to the invention.

When a microorganism is transferred to a new growth medium, usually a lag phase occurs when no immediate increase in cell numbers or cell mass takes place. The lag phase varies substantially in time depending on the type and state of the microorganism, and the contents of the former environment and the new culture medium. After the lag phase, the microorganism enter what is called the exponential or log phase, when the microorganisms are growing and dividing at the maximal rate possible given their potential, the nature of the medium and other culture conditions. After the exponential phase, the microorgansims enter a stationary phase when population growth ceases and the growth curve becomes horizontal. After the stationary phase, detrimental environmental changes like nutrient deprivation and accumulation of waste products lead to a death phase.

The present invention relies on monitoring growth of microorganisms by measuring the change in glucose concentration in the culture medium over time, in the presence or absence of an antibiotic agent. As this change is most significant during the exponential phase, the microorgansims are initially cultivated in a reference culture medium, comprising glucose and not comprising the antibiotic agent, for a period of time sufficient to observe a decrease in glucose concentration in the reference culture medium. When the decrease in glucose concentration in the reference culture medium is observed, it is considered that the culture has entered the exponential growth phase and that it is adequate to perform the other steps of the method.

The cultivation of microorgansims according to the present invention may be performed in any type of container that is suitable for such cultivation. It is presently preferred to perform the cultivation in liquid culture medium, and suitable containers are thus adapted to hold liquid culture medium. In order to automate the method and reduce the footprint of an automated system running the method, cultivation in microtiterplates is presently preferred. Microtiterplates for cultivation of microorganisms are commercially available from a wide range of suppliers, e.g. Greiner Bio-One (Kremsmünster, Austria).

Cultivation of microorganisms is standard practice for the person of ordinary skill in the art and described in numerous textbooks in the field, i.a. the book series "Methods in Microbiology" published by Academic Press.

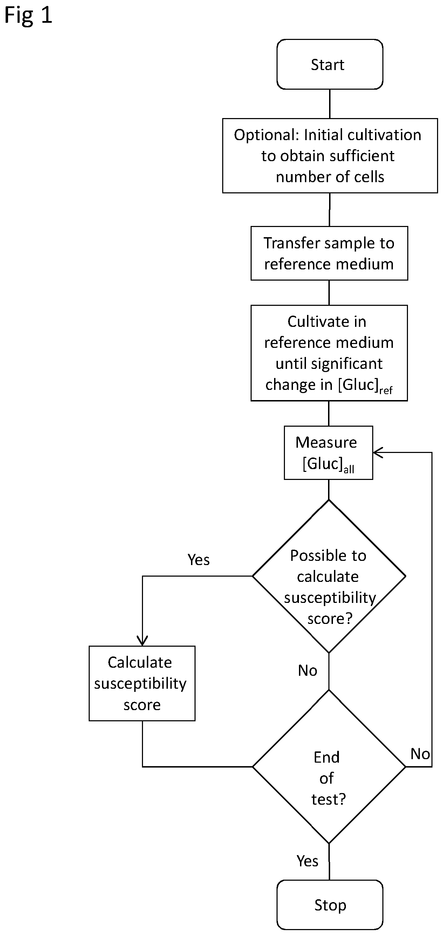

One aspect of the invention will now be described with reference to the flow diagram of FIG. 1, which illustrates a method for analysing a glucose metabolizing microorganism's susceptibility to an antibiotic agent by means of glucose measurement.

If necessary, a sample such as a blood or other body fluid sample obtained from a subject is subjected to initial cultivation in a reference culture medium (i.e. a culture medium not comprising antibiotic agent) in order to obtain a sufficient amount of viable microorganisms.

If it is considered adequate, the sample comprising microorganisms (that may or may not have been subjected to initial cultivation) is diluted with culture medium to yield a concentration of about 10⁵-10⁷ cfu/mL culture medium.

In the method, the microorganisms are then cultivated in a plurality of culture medium containers, comprising at least one reference culture medium container comprising no antibiotic agent and at least one culture medium container comprising an antibiotic agent.

A sample, or a part of a sample, is cultivated in a container comprising a culture medium comprising glucose, but no antibiotic agent. This culture medium comprising no antibiotic agent is termed reference culture medium in the present disclosure.

In one embodiment, aliquots of the sample are initially transferred to the plurality of culture medium containers before cultivation in a reference culture medium container as described. The microorganisms are thus at the same time cultivated in the culture medium containers comprising antibiotic agent and in the reference culture medium container not comprising antibiotic agent.

In another embodiment, the entire sample is transferred to a reference culture medium container for cultivation. This could correspond to the initial cultivation as described above. When a decrease in glucose concentration in this reference culture medium is observed, aliquots of this reference culture medium are transferred to the at least one reference culture medium container and at least one culture medium container comprising antibiotic agent.

The glucose concentration in the reference culture medium (denoted [Gluc]_{ref} in Fig 1) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

The microorgansims are cultivated in the reference culture medium for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

If aliquots of the sample have not yet been transferred to the plurality of culture medium containers, at least one aliquot of the cultivated reference culture medium is transferred to a container comprising culture medium comprising glucose and antibiotic agent at a predetermined concentration, and at least one aliquot of the cultivated reference culture medium is transferred to a container comprising reference culture medium.

The glucose concentrations in all (i.e. both the containers comprising antibiotic agent and reference medium) the culture medium containers (denoted [Gluc]ₐₗₗ in Fig 1) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

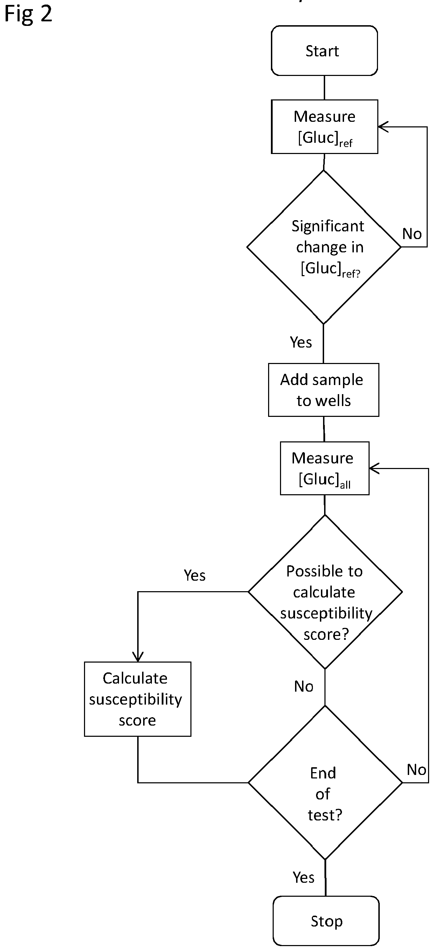

A further, more specific, embodiment of the method is disclosed in Fig 2.

In this embodiment, the method is applicable i.a. to analysis of blood samples from patients suspected of suffering from sepsis. Sepsis is a life threatening condition and mortality increases substantially with every hour that treatment is delayed. It is thus of utmost importance for a treating physician to be able to as soon as possible have information on what type of antibiotic compounds to use in treatment of the patient, and also the plasma concentrations needed to combat the infection.

In the present embodiment, a blood sample is obtained or provided and transferred to a blood culture bottle. The culture medium in the blood culture bottle then corresponds to the reference culture medium as described above, comprising glucose and no antibiotic agent.

According to standard procedures, two 10 mL samples may be obtained and transferred to one blood culture bottle for aerobic culture and one blood culture bottle for anaerobic culture. The blood culture bottles are then incubated at 37°C and gentle agitation. It is also possible to transfer a sample from the inoculated blood culture bottle to a culture medium container as used in the subsequent steps of the method and incubate the culture medium container under corresponding conditions.

The microorgansims are initially cultivated in the reference culture medium for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

The glucose concentration in the reference culture medium (denoted [Gluc]_{ref} in Fig 2) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

At least one aliquot of the blood culture medium is transferred to a reference culture medium container comprising no antibiotic agent.

At least one further aliquot of the sample is transferred to a container comprising antibiotic agent in a predetermined amount, yielding a predetermined concentration of the antibiotic agent in the container.

The glucose concentrations in all (i.e. both the containers comprising antibiotic agent and reference medium) the culture medium containers (denoted [Gluc]ₐₗₗ in Fig 2) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed if necessary until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

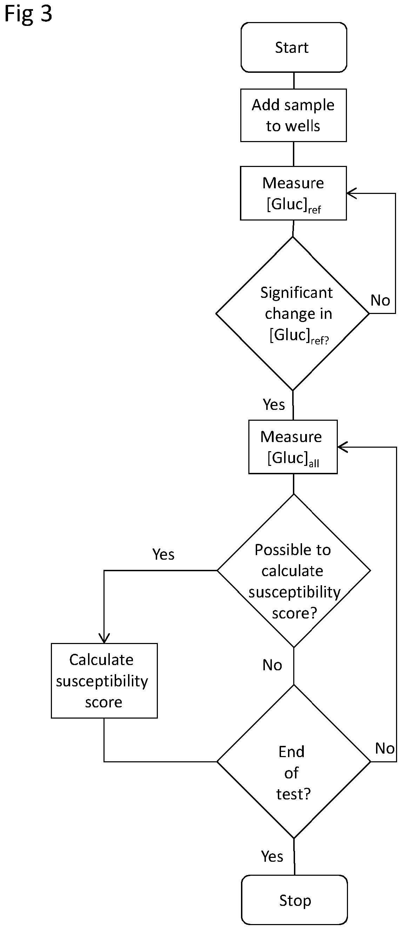

A further embodiment of the method is disclosed in Fig 3.

In this embodiment, the method is applicable i.a. to analysis of samples having a higher initial concentration of colony forming units of microorganisms, which reduces the time needed for cultivation.

In the present embodiment, aliquots of the sample are transferred to at least one container comprising reference culture medium, and at least one container comprising culture medium comprising an antibiotic agent. In a presently preferred embodiment, an aliquot is transferred to a volume of culture medium that is 99 times the volume of the aliquot, e.g. 10 µl sample is transferred to 990 µl culture medium. If the sample is from a culture on solid medium, a 1 µl loop of microorganisms may be transferred to 1 mL of liquid culture medium, and this culture medium is then diluted 1:19 to yield a suitable concentration of microorganisms.

The microorgansims are cultivated in the culture medium containers for a period of time sufficient to observe a decrease in glucose concentration in the reference culture medium. When the decrease in glucose concentration in the reference culture medium is observed, it is considered that the culture has entered, or is about to enter, the exponential growth phase and that it is adequate to perform the other steps of the method.

The glucose concentration in the reference culture medium (denoted [Gluc]_{ref} in Fig 3) is measured continuously or at specified time intervals. The time intervals may be chosen dependent on a variety of factors, such as type of microorganism, origin of sample, type of culture medium, urgency of analysis etc., but is typically between 0.5 and 6 times per hour, such as every 10, 12, 15, 20, 30, 60 or 120 minutes.

The glucose concentrations in all (i.e. both the containers comprising antibiotic agent and reference medium) the culture medium containers (denoted [Gluc]ₐₗₗ in Fig 3) are then measured continuously or at specified time intervals as discussed above. A data series comprising glucose concentrations as a function of time is thus obtained for each culture medium container comprising a known concentration of antibiotic agent.

The so obtained data series can be used for calculating susceptibility scores, as further explained below. Additional glucose measurements are performed if necessary until enough data have been obtained to calculate a susceptibility score, or until one or more conditions for ending the test procedure are met. Exemplary ways to calculate susceptibility scores are further described below. Exemplary conditions for deciding whether to end the test procedure are also discussed below.

### Statistical significance

The present invention relies in part on measurements of parameters and comparisons of measured parameters, most notably measurement and comparisons of glucose concentrations. It is noted that all measurements come with a margin of error that depends on a number of factors, including limitations in the measurement methods.

When a comparison of parameter values is made in a method according to the invention, it is preferred to determine if any difference between the parameter values is statistically significant. If the difference between the values is not statistically significant, the values are considered as being equal for the purposes of the invention. Only if the difference is statistically significant are the values considered different. This applies *mutatis mutandis* to the situation where a comparison is made to assess whether a parameter value differs to a certain degree, such as a given percentage, from a comparator parameter value.

A difference is considered as statistically significant if the probability that the measurement method provides the obtained differing parameter values despite the true parameter values being equal is below a certain threshold. The threshold is usually set at 5% (p<0.05) but may be set lower, such as at 1%, 0.5%, 0.01%, 0.005% or even lower.

Methods for determining if a difference is statistically significant are well-known in the art of statistics. Algorithms and pre-defined functions for making the determination are included in all major software tools commonly used for managing scientific measurement data, such as MS Excel (Microsoft, USA). One applicable statistical test is the t-test.

### Calculation of susceptibility scores

A susceptibility score can be qualitative, semi-quantitative, or quantitative.

In one embodiment, the susceptibility score is a qualitative susceptibility score classifying the microorganism as "susceptible", or "non-susceptible" to an antibiotic agent at a specified concentration.

This could be calculated by comparing the rate of change in glucose concentrations over time in the reference culture medium and the culture medium containing antibiotic agent at the specified concentration, respectively. If the glucose concentration decreases more rapidly in the reference culture medium as compared to the culture medium containing antibiotic agent, then the microorganism is susceptible to the antibiotic agent and the susceptibility score is set to "susceptible". If, on the other hand, the glucose concentration decreases at the same or substantially the same rate in the reference culture medium as in the culture medium containing antibiotic agent, then the microorganism is not susceptible to the antibiotic agent and the susceptibility score is set to "non-susceptible".

In one embodiment, the assignment of the score "susceptible" is conditioned on the rate of change in glucose concentration at the specified concentration of antibiotic agent being at, or below, a threshold fraction of the rate of change of glucose concentration in the reference medium. That is, the rate of change in the presence of the antibiotic agent should no more than for example half the rate of change in the absence of the antibiotic. The exact value of the threshold fraction of rate of change may be set by the skilled person in implementing the method for various purposes, and examples include 10%, 25%, 50%, 75%.

In one embodiment, the assignment of the score "non-susceptible" is made if the microorganism cannot be assigned the score "susceptible" at the relevant antibiotic concentration.

In one embodiment, the microorganism is cultivated in a plurality of containers together containing a serial dilution of an antibiotic agent, as well as in the absence of the antibiotic agent. Typically such a serial dilution is comprised of eight twofold dilutions expected to cover at least one concentration where the microorganism is susceptible to the antibiotic activity of the agent, e.g. 8.0, 4.0, 2.0, 1.0, 0.5, 0.25, 0.125, and 0.0625 µg/mL as commonly used for vancomycin for use against *E.coli.*

The susceptibility score could then be set to "susceptible" for all concentrations of antibiotic where the rate of change is below the threshold for designating the microorganism as "susceptible", and the susceptibility score is set to "non-susceptible" for the other concentrations.

In one embodiment, the susceptibility score is a qualitative susceptibility score classifying the microorganism as "susceptible", "intermediate" or "resistant" to an antibiotic agent at a specified concentration. The microorganism may then be designated as "resistant" if the rate of change in glucose concentration is substantially the same when cultivated with and without antibiotic agent, or if the rate of change in glucose concentration in the presence of antibiotic agent is above a predetermined threshold fraction of the rate of change in glucose concentration in the reference medium, e.g. above 90% of the rate of change in glucose concentration in the reference medium.

If the rate of change in glucose concentration is below an absolute threshold, i.e. the microorganism does not consume any substantial amounts of glucose or consume glucose at a low rate, then the microorganism is classified as "susceptible" to the antibiotic agent at that concentration.

The microorganism may be designated as "intermediate" if it cannot be classified as "resistant" or "susceptible".

A susceptibility score may also be quantitative.

In one embodiment, the susceptibility score is defined as the lowest concentration of antibiotic agent at which the growth is inhibited (Minimum Inhibitory Concentration, MIC) or the lowest concentration of antibiotic agent at which the microorganism is killed (Minimum Lethal Concentration, MLC).

In order to establish a MIC value, the microorganism is cultivated in a plurality of containers together containing a serial dilution of an antibiotic agent, as well as in the absence of the antibiotic agent, as generally described above. A "blank" container comprising culture medium, but no microorganism and no antibiotic is preferably also provided.

The MIC is the lowest concentration of antibiotic agent that results in a rate of change in glucose concentration that is significantly lower than in the reference culture medium. In various embodiments, the MIC is defined as the lowest concentration of antibiotic agent that results in a rate of change in glucose concentration that is equal to or less than a specified threshold value. The exact threshold value is likely to depend on a number of factors specific to the implementation of the method. The method can be calibrated and a suitable threshold value set by calibration against reference strains from e.g. American Type Culture Collection and comparison with reference MIC values, such as those published by the European Committee on Antimicrobial Susceptibility Testing, EUCAST (www.eucast.org). Purely by means of illustration, exemplary threshold values are 90%, 85%, 80%, 75%, 70%, 65%, 60%, 55%, 50%, 45%, 40%, 35%, or 30% of the rate of change in glucose concentration in the corresponding reference culture medium.

Normally, all concentrations of antibiotic agent that are higher than the MIC identified as above result in lower rates of decrease of change in glucose concentration than the MIC. If, on the contrary, several concentrations of antibiotic agent that are higher than the MIC lead to a higher decrease of the rate of change in glucose concentration, then it can be deduced that the antibiotic activity of the agent does not in itself impair the growth of the microorganism. The MIC value can then be disregarded.

A Minimum Lethal Concentration (MLC) can be defined as the lowest concentration of antibiotic agent that results in a rate of change in glucose concentration that is indicative of killing of the microorganism, i.e. the rate of change in glucose concentration is essentially zero or is less than a threshold fraction of the rate of change of glucose concentration in the reference culture medium, such as less than 25%, less than 20%, less than 15%, less than 10%, or less than 5% of the rate of change of glucose concentration in the reference culture medium.

Reference strains of various microorganisms with known susceptibilities to antibiotic agents are available from depositary institutions such as the American Type Culture Collection (ATCC). Such reference strains may be used when setting up a method according to the present invention in order to establish the correlations between rates of change of glucose concentrations and susceptibility scores as discussed in the current specification. The European Committee on Antimicrobial Susceptibility Testing (EUCAST) also publishes quality control tables comprising reference MIC values that can be used to calibrate the method.

### End of test conditions

The test procedure is terminated when at least one of the below conditions apply:
1. A susceptibility score as defined above can be established.
2. A time deemed to limit the test has passed.
3. No decrese in glucose concentration can be detected between one or more measurement occasions.

### Microorganisms

The method according to the invention may be used to analyse susceptibility of any glucose-metabolizing microorganism. Preferably, the microorganisms are human or animal pathogens, i.e. able to cause disease in an infected human or animal.

Exemplary species of microorganisms are provided below.

Bacteria: *Actinomyces israelii, Bacillus anthracis, Bacteroides fragilis, Bordetella pertussis, Borrelia burgdorferi, Borrelia garinii, Borrelia afzelii, Brucella abortus, Brucella canis, Brucella melitensis, Brucella suis, Campylobacter jejuni, Chlamydia pneumoniae, Chlamydia trachomatis, Chlamydophila psittaci, Clostridium botulinum, Clostridium difficile, Clostridium perifringens, Clostridium tetani, Corynebacterium diphtheriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira spp., Listeria monocytogenes, Mycobacterium leprae, Mycobacterium tuberculosis, Mycoplasma pneumoniae, Neisseria gonorrhoeae, Neisseria menigitidis, Pseudomonas aeruginosa, Nocardia asteroides, Rickettsia rickettsii, Salmonella typhi, Salmonella typhimurium, Salmonella spp. Shigella sonnei, Shigella dysenteriae, Staphylococcus aureus, Staphylococcus epidermidis, Staphylococcus saprophyticus, Streptococcus agalactieae, Streptococcus pneumoniae, Streptococcus pyogenes, Streptococcus viridans, Treponema pallidum, Vibrio cholerae, Yersinia pestis*

Fungi: *Candida albicans, Filobasidiella neoformans, Lenzites subferruginea, Penicillium ludwigii, Issatchenkia orientalis, Candida parapsilosis, Aspergillus flavus, Aspergillus fumigatus, Fusarium verticillioides, Hamigera insecticola, Aspergillus alabamensis, Scedosporium apiospermum, Scedosporium apiospermum, Fusarium solani, Trichophyton rubrum, Trichophyton interdigitale.*

Protozoa: *Entamoeba histolytica, Giardia lamblia, Cryptosporidium, Trichomonas vaginalis, Toxoplasma gondii, Plasmodium vivax, Plasmodium ovale, Plasmodium malariae and Plasmodium falciparum.*

### Measurement of glucose concentrations

A number of techniques for measuring glucose concentration in culture media are available and can be used in the present invention. A few of these are discussed further below.

### Microwave measurements

One apparatus useful in measuring glucose concentration in culture media uses microwaves. An illustrative glucose measurement apparatus comprises a sensor unit with an antenna configured to excite an electromagnetic wave in a measurement chamber. The measurement chamber is dimensioned to be in resonance for a predetermined frequency of the electromagnetic wave. The sensor unit further comprises a sample holder configured to hold the sample in a position relative the measurement chamber. The sample holder may be a through hole for a capillary extending through the measurement chamber, thus providing easy insertion of a liquid sample to the measurement chamber.

The apparatus further comprises a control unit, which comprises a tuneable oscillator and an amplifier for providing the antenna with a RF signal. The control unit is further configured to measure for example the return-loss from the measurement chamber, which provides a way to determine the resonance frequency of the measurement chamber. If a sample containing glucose is placed in the sample holder, the permittivity of the glucose will change the resonance frequency of the measurement chamber. By detecting the change of resonance frequency an indication of a changed glucose concentration in the sample is provided. It is important to notice that the control unit is mainly configured to detect a frequency difference; this means that no calibration of resonance frequency to glucose concentration is necessary as long as the measurements are performed with the same control unit.

### Enzyme electrodes

Enzyme electrodes generally comprise the enzyme glucose oxidase immobilized on a solid substrate. One such enzyme electrode was described already in 1984 (Cass et al., Anal. Chem. 1984, 56, 667-671).The electrode uses a substituted ferricinium ion as a mediator of electron transfer between immobilized glucose oxidase and a graphite electrode. A linear current response, proportional to a glucose concentration over 1-30 mM was observed.

Enzyme electrodes are commonly used to measure blood glucose levels, i.a. in monitoring of diabetes patients. Enzyme electrodes are usually used together with a read-out device adapted to transform the electrical current generated by the enzyme electrode into a glucose concentration value. One such device is the WaveSense Jazz glucometer commercially available from AgaMatrix (Salem, USA).

Enzyme electrodes configured for continuous measurement of glucose concentrations are also known in the art.

### Further technologies

It has also been suggested to use infrared spectroscopic methods such as attenuated total reflectance coupled with wavelet transformation (ATR-WT-IR) as a precise measurement of aqueous glucose concentrations (Al-Gharabli, Sensors (Basel). 2009; 9(8): 6254-6260).

### Applications of the method

The method according to the present invention is useful in a number of applications. One such application is assessing efficacy of a candidate antibiotic agent in treatment of in treatment of a medical condition caused or complicated by an infection caused by one or more microorganisms. Such medical conditions include e.g. septicaemia, pneumonia, endocarditis, peritonitis, and meningitis. These conditions may be life-threatening and a rapid determination of which antibiotic agent to use may be crucial to saving the patient's life.

### Experimental protocol

The following procedures illustrate in some detail one way of putting the invention into practice. The person skilled in the art will understand that various changes may be made to the protocols below without departing from the scope of the invention.

### Culture media based on Mueller Hinton Broth (MHB) for 96 well plate

Mueller Hinton broth generally comprises beef extract or beef infusion, casein hydrolysate and starch. The general proportions are 2 g/L beef extract or 300 g/L beef infusion, 17.5 g/L casein hydrolysate, and 1.5 g/L starch. The final pH is 7.3±0.2 at 25°C. (Mueller J. H. and Hinton J., 1941, Proc. Soc. Exp. Biol. Med., 48:330).

Steps that arrives at final mix in plates being 1mg/ml d-glucose and 100% MHB.

Antibiotic agents have been fixated at bottom of wells through evaporation and the amount in each well corresponds to the specified concentration as per standard when 0.1 ml of solution is added.

### Mueller Hinton broth

### A. Prepare suspension

1. Suspend 1 loop [1µl] of bacteria (cells) in 1ml MHB [100%]. Vortex until fully suspended
2. Label the test tube and put it in the micro tube stand.
3. Repeat from step 1 if many suspensions are needed.

### B. Prepare Culture Media Base

1. Transfer 0.0167 ml D-Glucose stock solution [300 mg/ml] to a 5 ml centrifuge tube containing 4,983 ml MHB [100%].
2. Vortex or swirl tube for about 10- 20 s
3. Repeat from step 1 for a second or more tube(s) of 5 ml is/are needed.

### C. Mix reference blank in the well plate (no cells no ab)

1. By pipette, transfer 0.1 ml Ascilion Media base to the designated blank on the 96 well plate.
2. Repeat from relevant step to obtain the number of blanks that you may need

### D. Mix sample solution, dispense and measure

1. Transfer 0.950 ml Culture Medium to a 1ml eppendorf tube and label it as sample "xxx", then put it in the microbe stand.
2. Vortex the test tube containing your cell suspension and then, by pipette, transfer 0.05 ml to the eppendorf tube labeled as sample "xxx" and and pipette up and down three times to ensure mixing
3. From the eppendorf tube labelled sample "xxx" transfer 0.1 ml to each well in the designated row for that particular sample as well as 0.1 ml to the designated reference well containing no antibiotic
4. Repeat the process for each sample tube.
5. Measure all wells, starting with the references.

### Culture media based on Brain Heart Infusion (BHI) for 96 well plate

Brain Heart Infusion broth generally comprises beef heart infusion, calf brain infusion, disodium hydrogen phosphate, D(+)-glucose, and peptone. The general proportions are beef heart (infusion from 250g), 5 g/L, calf brains (infusion from 200g), 12.5 g/L, disodium hydrogen phosphate, 2.5 g/L, D(+)-glucose, 2 g/L, peptone, 10 g/L. final pH is 7.4±0.2 at 25°C. The (SigmaAldrich cat. # 53286). In the present invention, BHI is used with a lower concentration of glucose.

Steps that arrives at final mix in wells being 1mg/ml d-glucose and 2% BHI

Antibiotic agents have been fixated at bottom of wells through evaporation and the amount in each well corresponds to the specified concentration as per standard when 0.1 ml of solution is added.

### A. Prepare Culture Media components

1. Directly in a centrifuge tube or in a glass beaker: Under stirring dissolve 1 g BHI dry powder without glucose in 25 ml of deionized water
2. Label tube "4% BHI". If dissolved in beaker transfer solution to centrifuge tube and label it as mentioned.
3. Prepare centrifuge tube with 10 ml of 2 mg/ml glucose solution: i.e transfer 0.067 ml of glucose stock solution [300mg/ml] to an eppendorf containing 9.933 ml of deionized water and label the tube "2 mg/ml GLU."

### B. Prepare suspension

1. Into two ependorf tube, transfer 1 ml of 4% BHI to the first and 0.5 ml of 4% BHI to the second along with 0.5 ml of deionized water.
2. In the first, suspend 1 loop [1µl] of bacteria. Vortex until fully suspended. Label "1:1" along with name of bacteria.
3. Transfer 0.1 ml from the 1:1 suspension above to the second eppendorf tube and pipette up and down three times. Label it "1:9 - 2%" along with name of bacteria.
4. Repeat from step 1 if many suspensions are needed.

### C. Prepare Culture Media for blanks and dispense for blank(s)

1. Transfer 5 ml of 4% BHI to a new centrifuge tube.
2. Then by transfer-pipette mix 5 ml from the tube labelled "2 mg/ml GLU." into the tube described above (C, step 1). Culture Media is now ready [ 2% BHI + 1mg/ml glucose]
3. Vortex or swirl tube for about 10- 20 s and label it "Culture Medium".
4. By pipette, transfer 0.1 ml Culture Medium to the designated blank(s) on the 96 well plate
5. Repeat from relevant step to obtain the number of blanks that you may need

### D. Mix sample solution, dispense and measure

1. Transfer 0.5 ml of Culture Medium to an eppedorf tube and label in "1:19"
2. After vortexing for 10s, transfer 0.5 ml of the cell suspension labelled "1:9 - 2%" to the test tube above (D, step 1) and label it additional as sample "xxx" - pipette up and down three times to ensure mixing
3. Now from the eppendorf tube you just labelled "1:19" and sample "xxx", transfer 0.1 ml to each well in the designated row for that particular sample as well as 0.1 ml to the designated reference well containing no antibiotic
4. Repeat the process for each sample tube.
5. Measure all wells, starting with the references.

The present invention is not limited to the above-described preferred embodiments or the examples provided below. Various alternatives, modifications and equivalents may be used. Therefore, the above embodiments should not be taken as limiting the scope of the invention, which is defined by the appended claims.

### Examples

### Example 1: Quantitative susceptibility score

Strains of *Escherichia coli* (ATCC 25922), *Enterococcus faecalis* (ATCC 29212) and *Staphylococcus aureus* (ATCC 29213) were inoculated in blood culture bottles (SVA, Uppsala, Sweden) and cultivated according to the manufacturer's instructions until positive.

Medium from each blood culture bottle was diluted 1:100 with cation adjusted Mueller-Hinton broth (SVA, Uppsala, Sweden) supplemented with glucose to a concentration of 1.0 mg/mL.

100 µL medium from blood culture bottles was added to wells of a microtitre plate comprising serial dilutions of the antibiotic agents vancomycin, gentamicin, and cefoxitin (all obtained from Sigma Aldrich), according to Table 1.

**Table 1: Concentrations of antibiotic agents are given in µg/mL. C=Cefoxitin; G=Gentamicin; V=Vancomycin; Ref=Bacteria with no antibiotic; Blank=Only medium; RBC; Red Blood Cells in medium**

| | 1 | 2 | 3 | 4 | 5 | 6 | 7 | 8 | 9 | 10 | 11 | 12 |
|---|---|---|---|---|---|---|---|---|---|---|---|---|
| A | | | | | | C 32 | | G 32 | | V 8 | | Ref |
| B | | | | | | C 16 | | G 16 | | V 4 | | Blank |
| C | | | | | | C 8 | | G 8 | | V 2 | | RBC |
| D | | | | | | C 4 | | G 4 | | V 1 | | |
| E | | | | | | C 2 | | G 2 | | V 0.5 | | |
| F | | | | | | C 1 | | G 1 | | V 0.25 | | |
| G | | | | | | C 0.5 | | G 0.5 | | V 0.125 | | |
| H | | | | | | C 0.25 | | G 0.25 | | V 0.0625 | | |

Glucose concentrations were measured at 0, 60, 120, 180, and 240 minutes using a WaveSense Jazz glucometer (AgaMatrix, Inc., Salem, NH, USA) with accompanying test strips. The glucose measurement device had a detection limit of 20 mg glucose per mL and no values below 20 mg/mL were recorded.

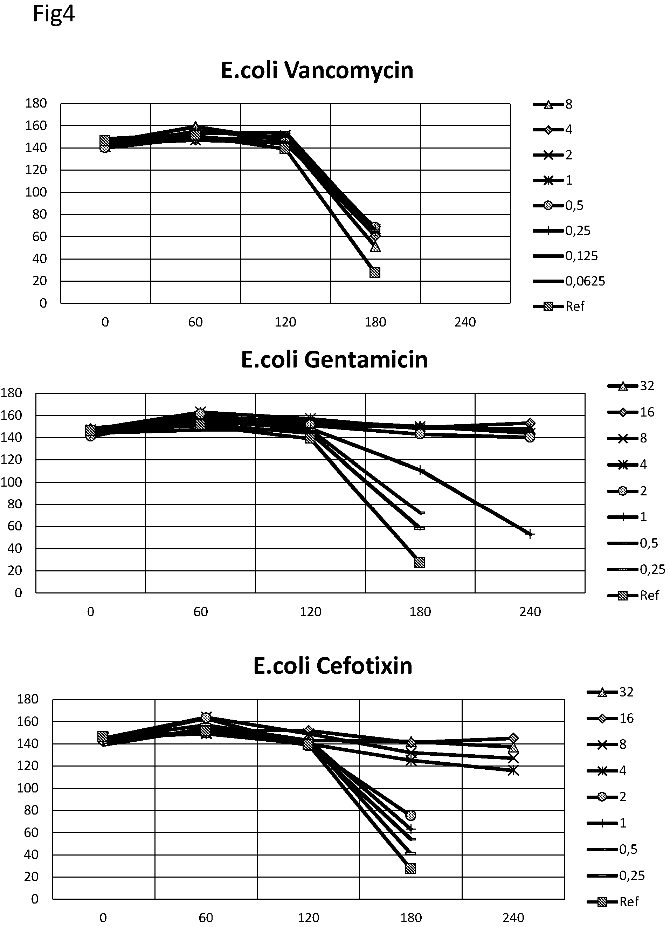

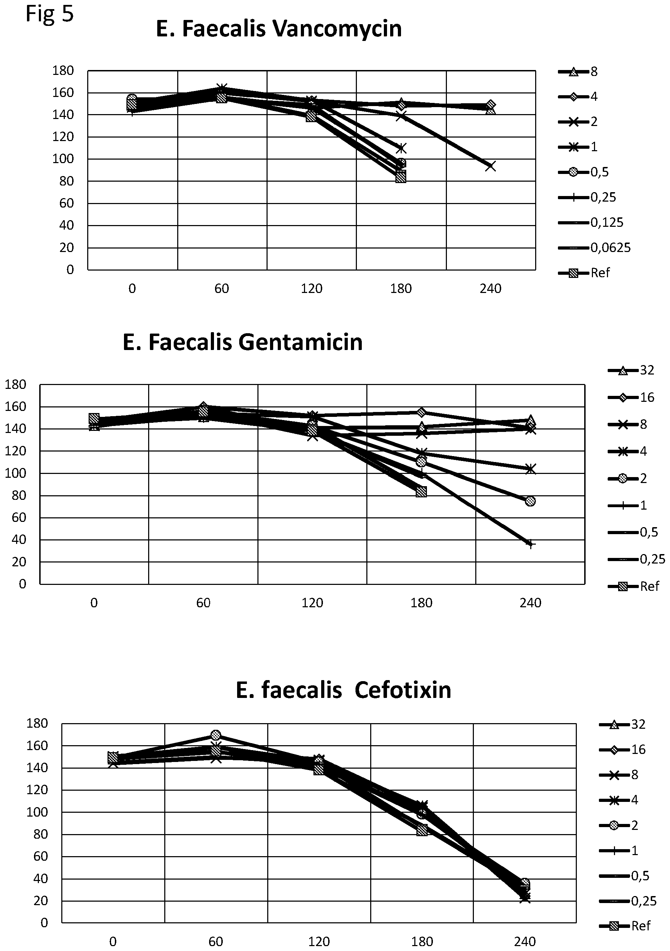

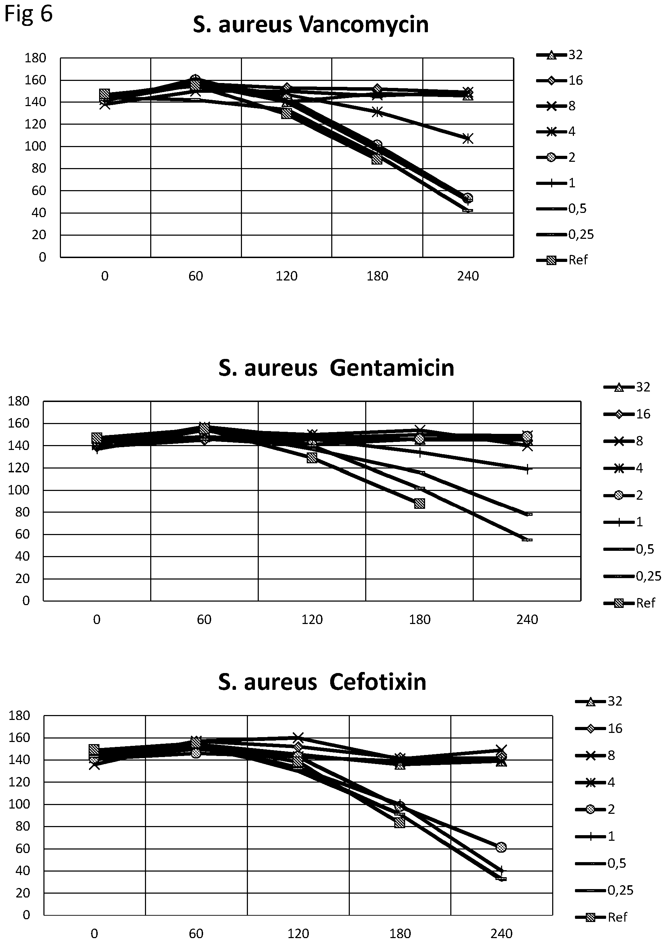

The results are shown in Tables 2, 3, 4 and figures 4, 5, and 6.

**Table 2: E.coli. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 146 | 150 | 143 | 142 | 137 | 3 | 5% |
| B6 | 16 | C | 142 | 152 | 152 | 141 | 145 | 4 | 6% |
| C6 | 8 | C | 145 | 164 | 149 | 132 | 127 | 11 | 18% |
| D6 | 4 | C | 146 | 149 | 140 | 125 | 116 | 12 | 19% |
| E6 | 2 | C | 143 | 163 | 138 | 75 | | 63 | 102% |
| F6 | 1 | C | 144 | 150 | 143 | 63 | | 80 | 129% |
| G6 | 0,5 | C | 139 | 154 | 140 | 54 | | 86 | 139% |
| H6 | 0,25 | C | 145 | 157 | 143 | 41 | | 102 | 165% |
| | | | | | | | | | |
| A8 | 32 | G | 148 | 158 | 155 | 150 | 147 | 4 | 6% |
| B8 | 16 | G | 143 | 155 | 152 | 149 | 153 | 1 | 1% |
| C8 | 8 | G | 144 | 156 | 153 | 150 | 144 | 5 | 7% |
| D8 | 4 | G | 147 | 163 | 157 | 148 | 148 | 5 | 7% |
| E8 | 2 | G | 141 | 161 | 151 | 143 | 140 | 6 | 9% |
| F8 | 1 | G | 144 | 155 | 148 | 111 | 53 | 48 | **77%** |
| G8 | 0,5 | G | 144 | 147 | 147 | 72 | | 75 | 121% |
| H8 | 0,25 | G | 149 | 152 | 144 | 58 | | 86 | 139% |
| | | | | | | | | | |
| A10 | 8 | V | 145 | 159 | 147 | 51 | | 54 | 87% |
| B10 | 4 | V | 145 | 147 | 145 | 61 | | 43 | 69% |
| C10 | 2 | V | 144 | 147 | 151 | 67 | | 40 | 65% |
| D10 | 1 | V | 141 | 155 | 149 | 65 | | 45 | 73% |
| E10 | 0,5 | V | 140 | 149 | 145 | 68 | | 41 | 65% |
| F10 | 0,25 | V | 146 | 150 | 145 | 66 | | 42 | 68% |
| G10 | 0,125 | V | 148 | 153 | 154 | 65 | | 44 | 71% |
| H10 | 0,0625 | V | 143 | 148 | 144 | 65 | | 42 | 67% |
| A12 | - | no ab | 146 | 151 | 139 | 27 | | 62 | 100% |
| B12 | - | no ab | 145 | 151 | 160 | 151 | 155 | | |
| C12 | - | no ab | 149 | 160 | 168 | 154 | 162 | | |

**Table 3: E. faecalis. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 148 | 155 | 140 | 102 | 27 | 27 | 74% |
| B6 | 16 | C | 148 | 154 | 148 | 105 | 25 | 25 | 68% |
| C6 | 8 | C | 144 | 149 | 147 | 104 | 23 | 23 | 63% |
| D6 | 4 | C | 150 | 159 | 146 | 106 | 27 | 27 | 74% |
| E6 | 2 | C | 149 | 169 | 145 | 98 | 36 | 36 | 99% |
| F6 | 1 | C | 148 | 159 | 142 | 98 | 31 | 31 | 85% |
| G6 | 0,5 | C | 145 | 150 | 144 | 87 | 32 | 32 | 88% |
| H6 | 0,25 | C | 151 | 155 | 138 | 88 | 34 | 34 | 93% |
| | | | | | | | | | |
| A8 | 32 | G | 143 | 151 | 141 | 142 | 148 | 1 | 3% |
| B8 | 16 | G | 147 | 160 | 152 | 155 | 141 | 6 | 18% |
| C8 | 8 | G | 143 | 155 | 134 | 136 | 140 | 5 | 14% |
| D8 | 4 | G | 147 | 154 | 151 | 118 | 104 | 17 | 46% |
| E8 | 2 | G | 147 | 156 | 143 | 110 | 75 | 27 | **75%** |
| F8 | 1 | G | 145 | 150 | 138 | 100 | 36 | 38 | 106% |
| G8 | 0,5 | G | 146 | 160 | 138 | 97 | | 53 | 148% |
| H8 | 0,25 | G | 142 | 156 | 143 | 87 | | 52 | 144% |
| | | | | | | | | | |
| A10 | 8 | V | 150 | 156 | 146 | 151 | 145 | 3 | 7% |
| B10 | 4 | V | 147 | 162 | 153 | 148 | 149 | 7 | 19% |
| C10 | 2 | V | 147 | 160 | 152 | 139 | 94 | 11 | 29% |
| D10 | 1 | V | 151 | 164 | 153 | 110 | | 27 | **75%** |
| E10 | 0,5 | V | 154 | 155 | 148 | 96 | | 30 | 82% |
| F10 | 0,25 | V | 143 | 155 | 146 | 96 | | 30 | 82% |
| G10 | 0,125 | V | 148 | 154 | 149 | 94 | | 30 | 83% |
| H10 | 0,0625 | V | 145 | 156 | 139 | 89 | | 34 | 93% |
| A12 | - | no ab | 149 | 155 | 138 | 83 | | 36 | 100% |
| B12 | - | no ab | 152 | 166 | 155 | 167 | | | |
| C12 | - | no ab | 150 | 160 | 153 | 155 | | | |

**Table 4: S. aureus. C=Cefoxitin, G=Gentamicin, and V=Vancomycin. F=the rate of change in glucose concentration in relation to the reference.**

| ***Wells*** | ***[Ab]*** | ***Antibiotic*** | ***t=0*** | ***t=60*** | ***t=120*** | ***t=180*** | ***t=240*** | ***dC*/*dT*** | ***F*** |
|---|---|---|---|---|---|---|---|---|---|
| A6 | 32 | C | 145 | 154 | 145 | 136 | 139 | 9 | 27% |
| B6 | 16 | C | 140 | 157 | 152 | 142 | 142 | 8 | 22% |
| C6 | 8 | C | 136 | 157 | 160 | 141 | 149 | 8 | 24% |
| D6 | 4 | C | 142 | 150 | 143 | 139 | 139 | 6 | 16% |
| E6 | 2 | C | 141 | 146 | 143 | 98 | 61 | 24 | **72%** |
| F6 | 1 | C | 147 | 155 | 133 | 100 | 40 | 28 | 82% |
| G6 | 0,5 | C | 144 | 155 | 130 | 91 | 32 | 32 | 96% |
| H6 | 0,25 | C | 143 | 152 | 132 | 91 | 33 | 31 | 91% |
| | | | | | | | | | |
| A8 | 32 | G | 144 | 148 | 145 | 146 | 145 | 1 | 3% |
| B8 | 16 | G | 139 | 145 | 150 | 145 | 145 | 0 | 0% |
| C8 | 8 | G | 142 | 154 | 150 | 154 | 140 | 0 | 0% |
| D8 | 4 | G | 141 | 157 | 147 | 150 | 149 | 4 | 10% |
| E8 | 2 | G | 144 | 154 | 141 | 146 | 148 | 4 | 12% |
| F8 | 1 | G | 137 | 154 | 146 | 134 | 119 | 10 | 30% |
| G8 | 0,5 | G | 143 | 154 | 137 | 116 | 78 | 19 | 57% |
| H8 | 0,25 | G | 143 | 146 | 140 | 102 | 55 | 22 | **66%** |
| | | | | | | | | | |
| A10 | 32 | V | 145 | 157 | 140 | 148 | 146 | 5 | 13% |
| B10 | 16 | V | 146 | 157 | 153 | 152 | 149 | 3 | 7% |
| C10 | 8 | V | 138 | 150 | 150 | 146 | 149 | 2 | 6% |
| D10 | 4 | V | 142 | 157 | 147 | 131 | 107 | 13 | **39%** |
| E10 | 2 | V | 142 | 160 | 143 | 101 | 53 | 30 | 88% |
| F10 | 1 | V | 141 | 161 | 140 | 97 | 51 | 32 | 96% |
| G10 | 0,5 | V | 142 | 156 | 142 | 100 | 51 | 28 | 84% |
| H10 | 0,25 | V | 144 | 142 | 133 | 92 | 42 | 25 | 75% |
| A12 | - | no ab | 147 | 155 | 129 | 88 | | 34 | 100% |
| B12 | - | no ab | 149 | 148 | 151 | 153 | | | |
| C12 | - | no ab | 147 | 161 | 160 | 163 | | | |

### Calculation of susceptibility score

Susceptibility scores corresponding to MIC values were calculated as described above. A 80% threshold was applied, i.e. the lowest concentration of antibiotic that entailed a rate of change in glucose concentration that was equal to or less than 80% of the reference was selected as the Minimal Inhibitory Concentration. The rate of change was calculated between the measurements at 60 and 180 minutes. For vancomycin and *E.coli,* and cefoxitin and E.faecalis, MIC values of 0.0625 and 4 µg/mL, respectively, were initially obtained. However, as the rate of decrease in glucose concentration was higher for the the concentrations 8 and 32 µg/mL, respectively, it was found that the initial MIC values should be disregarded.

The results are disclosed in Table 5. The total time for obtaining the MIC values were 4 hours for all three microorganisms.

For comparison, the results were compared to reference MIC values published by the European Committee on Antimicrobial Susceptibility Testing (EUCAST) in "Routine and extended internal quality control for MIC determination and disk diffusion as recommended by EUCAST" Version 7.0, 2017.(available from http://www.eucast.org) for the same strains, *Escherichia coli* ATCC 25922, *Staphylococcus aureus* ATCC 29213, and *Enterococcus faecalis* ATCC 29212.

**Table 5. All MIC values are in ng/mL. N/A: The microorganism is not susceptible to the antibiotic agent and no MIC value can be calculated.**

| | Vancomycin | | Gentamicin | | Cefoxitin | |
|---|---|---|---|---|---|---|
| | Measured | EUCAST | Measured | EUCAST | Measured | EUCAST |
| *E.coli* | N/A | N/A | 1 | 0.5 (0.25-1) | 4 | 4(2-8) |
| *E.faecalis* | 1 | 2 (1-4) | 2 | 8 (4-16) | N/A | N/A |
| *S.aureus* | 4 | 1 (0.5-2) | 0.25 | 0.25-0.5 (0.125-1) | 2 | 2 (1-4) |

The EUCAST MIC values are obtained by disk diffusion methodology using Mueller-Hinton agar, 35±1ºC, incubation time 18±2h. The ranges in parentheses were obtained (by EUCAST) from International Standards Organisation, ISO 20776-1: 2006.

The MIC values obtained by the method thus generally corresponds well with reference values published in the generally accepted quality control tables. The values could be obtained in just 4 hours, as compared to 16-20 hours using standard prior art technology.

### Example 2: Qualitative susceptibility score

If the susceptibility score is a qualitative susceptibility score classifying the microorganism as "susceptible", or "non-susceptible" to an antibiotic agent the answer could be given in as little as 1 hour.

Bacteria from plated cultures were suspended in cation adjusted Mueller-Hinton broth (SVA, Uppsala, Sweden) supplemented with glucose. Consequently, the concentration of microorgansims was typically two orders of magnitude higher as compared to Example 1. The concentrations of antibiotic agents were also increased as compared to Example 1. The tested microorganisms was one strain of *E. faecium,* two strains of *E.coli* and one *S*. *aureus.*

**Table 6. "--" denotes no measurement**

| **Bacteria** | **[Ab] µg/mL** | **Antibiotic** | **t=0** | **t=15** | **t=30** | **t=45** |
|---|---|---|---|---|---|---|
| *E. faecium* | 256 | Gentamicin | 79 | 47 | -- | - |
| *E. coli 17* | 256 | Gentamicin | -- | 131 | 126 | 123 |
| *E. coli ATCC* 25922 | 256 | Gentamicin | -- | 156 | 155 | 136 |
| *S. aureus* | 256 | Gentamicin | -- | 137 | 139 | 131 |

The results in table 6 shows that *E. faecium* is non-susceptible towards Gentamicin and the two *E. coli* strains and the *S*. *aureus* are all "susceptible".

With slightly lower microbe concentrations e.g. directly from positive BC-bottles (^{~}10⁹ CFU/ml) the results are shown at least within 2 hours, where *E. coli* ATCC 25922is classified as susceptible and *E. faecium* is non-susceptible. The results are shown in Table 7.

**Table 7. "--" denotes no measurement**

| ***Wells*** | **[Ab] µg/mL** | ***Antibiotic*** | **t=0** | **t=15** | **t=30** | **t=45** | **t=60** | **t=75** | **t=90** | **t=105** |
|---|---|---|---|---|---|---|---|---|---|---|
| *E. coli* ATCC 25922 | - | - | 144 | 137 | 130 | 101 | 73 | 42 | -- | - |
| *E. faecium* | - | - | 146 | 156 | 148 | 144 | 136 | 122 | 94 | 55 |
| Blank | - | - | 141 | 152 | 154 | 154 | 156 | 153 | 158 | 160 |
| *E. faecium* | 256 | Gentamicin | 144 | -- | -- | 105 | 77 | 47 | | |
| *E. coli* ATCC 25922 | 256 | Gentamicin | 145 | -- | -- | 155 | 151 | 141 | 140 | 135 |

## Claims

1. A method for analysing susceptibility of a glucose metabolizing microorganism to at least one antibiotic agent, comprising
a) Cultivating said glucose metabolizing microorganism in a reference culture medium, comprising glucose and not comprising the antibiotic agent; and measuring the glucose concentration in the reference culture medium to obtain time-resolved glucose concentration data, wherein said cultivation extends for a period of time sufficient to observe a decrease in glucose concentration in the reference culture medium;
b) Simultaneously cultivating said glucose metabolizing microorganism in a culture medium contained in a culture medium container, in the presence of a predetermined concentration of an antibiotic agent and in the presence of glucose;
c) Measuring the concentration of glucose in the culture medium in said culture medium container at least when the glucose concentration in the reference culture medium has decreased below a predetermined threshold; and
d) Calculating a susceptibility score reflecting the susceptibility of the glucose metabolizing microorganism to the antibiotic agent based on a relation between the predetermined concentration of the agent with antibiotic activity, the glucose concentration data obtained in step c), and the time-resolved glucose concentration data obtained in step a).

2. The method according to claim 1, wherein step c) comprises obtaining time-resolved glucose concentration data for the culture medium in the culture medium container during at least a part of the time period during which the glucose metabolizing microorganism is cultured.

3. The method according to any one of claims 1-2, further comprising obtaining time-resolved glucose concentration reference data for culture medium in a reference container comprising no glucose metabolizing microorganism.

4. The method according to any one of claims 1-3, wherein said glucose metabolizing microorganism is cultured in a plurality of culture medium containers and wherein said antibiotic agent is present in each culture medium container in an amount that is predetermined for each culture medium container, optionally wherein the plurality of culture medium containers together contain a series of concentrations of the antibiotic agent.

5. The method according to any one of claims 1-4, wherein said glucose metabolizing microorganism is cultivated in a plurality of culture medium containers and wherein the culture medium containers together contain a plurality of different antibiotic agents.

6. The method according to any one of claims 1-5, wherein the susceptibility score is a qualitative susceptibility score, such as a qualitative susceptibility score defined for a specific concentration of a specific agent with antibiotic activity.

7. The method according to any one of claims 1-5, wherein the susceptibility score is a quantitative susceptibility score, such as a minimum inhibitory concentration (MIC), or a minimum lethal concentration (MLC).

8. The method according to claim 7, wherein the susceptibility score is calculated based on time-resolved glucose concentration data from a plurality of culture medium containers comprising the microorganism and together comprising a serial dilution of the antibiotic agent, a reference culture medium container comprising the microorganism and not comprising the antibiotic agent, and a blank culture medium container not comprising the microorganism and not comprising the antibiotic agent.

9. The method according to any one of the preceding claims, wherein measurement of glucose concentration involves a frequency measurement indicative of the glucose concentration optionally wherein the frequency measurement is a measurement of a resonance frequency of the culture medium container and/or wherein the frequency measurement is an electrical high frequency measurement.

10. The method according to any one of claims 1-8, wherein measurement of glucose concentration is performed with an enzyme electrode.

11. The method according to any one of claims 1-10, wherein the glucose concentration is measured within the culture medium container.

12. The method according to any one of claims 1-11, wherein the culture medium and reference culture medium is Mueller-Hinton broth or Brain Heart Infusion, supplemented with glucose.

13. A method for assessing efficacy of a candidate antibiotic agent in treatment of a medical condition caused or complicated by an infection, comprising providing a body fluid sample from a subject known or suspected of suffering from an infection and obtaining a susceptibility score reflecting the susceptibility of any glucose metabolizing microorganism present in said sample to said candidate antibiotic agent using a method according any one of claims 1-12, wherein the susceptibility score indicates the efficacy of the candidate antibiotic agent in the treatment of the medical condition, such as a medical condition selected from the group consisting of septicaemia, pneumonia, endocarditis, peritonitis, and meningitis.

14. Use of a liquid culture medium comprising Brain Heart Infusion or Mueller-Hinton broth comprising glucose at a concentration of 1 mg glucose per mL culture medium, in a method according to any one of claims 1-13.

## Patentansprüche

1. Verfahren zum Analysieren der Empfindlichkeit eines Glukose-metabolisierenden Mikroorganismus gegenüber mindestens einem antibiotischen Mittel, umfassend
a) Kultivieren des Glukose-metabolisierenden Mikroorganismus in einem Referenzkulturmedium, umfassend Glukose und nicht umfassend das antibiotische Mittel; und Messen der Glukosekonzentration in dem Referenzmedium zum Erhalten zeitaufgelöster Glukosekonzentrationsdaten, wobei sich das Kultivieren über einen Zeitraum erstreckt, der ausreichend ist, um eine Senkung der Glukosekonzentration in dem Referenzkulturmedium zu beobachten;
b) gleichzeitiges Kultivieren des Glukose-metabolisierenden Mikroorganismus in einem in einem Kulturmediumbehälter enthaltenen Kulturmedium in Gegenwart einer vorgegebenen Konzentration eines antibiotischen Mittels und in Gegenwart von Glukose;
c) Messen der Konzentration von Glukose in dem Kulturmediumbehälter, zumindest in dem Fall, wo die Glukosekonzentration in dem Referenzkulturmedium unter einen vorgegebenen Schwellenwert gefallen ist; und
d) Berechnen eines Empfindlichkeit-Scores, der die Empfindlichkeit des Glukose-metabolisierenden Mikroorganismus gegenüber dem antibiotischen Mittel basierend auf einem Verhältnis zwischen der vorgegebenen Konzentration des Mittels mit antibiotischer Aktivität, den im Schritt c) erhaltenen Glukosekonzentrationsdaten und den im Schritt a) erhaltenen zeitaufgelösten Glukosekonzentrationsdaten wiederspiegelt.

2. Verfahren nach Anspruch 1, wobei der Schritt c) das Erhalten zeitaufgelöster Glukosekonzentrationsdaten für das in dem Kulturmediumbehälter befindliche Kulturmedium während zumindest eines Teils der Zeitdauer, während welcher der Glukose-metabolisierende Mikroorganismus kultiviert wird, umfasst.

3. Verfahren nach einem der Ansprüche 1-2, ferner umfassend das Erhalten zeitaufgelöster Glukosekonzentrationsreferenzdaten für Kulturmedium in einem Referenzbehälter, der keinen Glukose-metabolisierenden Mikroorganismus enthält.

4. Verfahren nach einem der Ansprüche 1-3, wobei der Glukose-metabolisierende Mikroorganismus in mehreren Kulturmediumbehältern kultiviert wird, und wobei das antibiotische Mittel in jedem Kulturmediumbehälter in einer Menge vorhanden ist, die für jeden Kulturmediumbehälter vorbestimmt ist, wahlweise wobei die mehreren Kulturmediumbehälter insgesamt eine Reihe von Konzentrationen des antibiotischen Mittels enthalten.

5. Verfahren nach einem der Ansprüche 1-4, wobei der Glukose-metabolisierende Mikroorganismus in mehreren Kulturmediumbehältern kultiviert wird, und wobei die Kulturmediumbehälter insgesamt eine Mehrzahl von verschiedenen antibiotischen Mitteln enthalten.

6. Verfahren nach einem der Ansprüche 1-5, wobei der Empfindlichkeit-Score ein qualitativer Empfindlichkeit-Score ist, wie beispielsweise ein qualitativer Empfindlichkeit-Score, der für eine spezifische Konzentration eines spezifischen Mittels mit antibiotischer Aktivität definiert ist.

7. Verfahren nach einem der Ansprüche 1-5, wobei der Empfindlichkeit-Score ein quantitativer Empfindlichkeit-Score, wie beispielsweise eine minimale Hemmkonzentration (MHK) oder eine minimale lethale Konzentration (MLC), ist.

8. Verfahren nach Anspruch 7, wobei der Empfindlichkeit-Score basierend auf zeitaufgelösten Glukosekonzentrationsdaten aus einer Mehrzahl von Kulturmediumbehältern umfassend den Mikroorganismus und insgesamt umfassend eine Serienverdünnung des antibiotischen Mittels, einem Referenzkulturmediumbehälter umfassend den Mikroorganismus und nicht umfassend das antibiotische Mittel, und einem leeren Kulturmediumbehälter nicht umfassend den Mikroorganismus und nicht umfassend das antibiotische Mittel berechnet wird.

9. Verfahren nach einem der vorgehenden Ansprüche, wobei das Messen der Glukosekonzentration eine Frequenzmessung umfasst, die die Glukosekonzentration anzeigt, wahlweise wobei die Frequenzmessung eine Messung einer Resonanzfrequenz des Kulturmediumbehälters ist, und/oder wobei die Frequenzmessung eine elektrische Hochfrequenzmessung ist.

10. Verfahren nach einem der Ansprüche 1-8, wobei die Messung der Glukosekonzentration mit einer Enzymelektrode durchgeführt wird.

11. Verfahren nach einem der Ansprüche 1-10, wobei die Glukosekonzentration innerhalb des Kulturmediumbehälters gemessen wird.

12. Verfahren nach einem der Ansprüche 1-11, wobei das Kulturmedium und das Referenzkulturmedium Mueller-Hinton-Brühe oder Hirn-Herz-Infusion, mit Glukose ergänzt, ist.

13. Verfahren zum Beurteilen der Wirksamkeit eines antibiotischen Kandidatenmittels bei der Behandlung eines Gesundheitszustandes, der durch eine Infektion verursacht oder kompliziert worden ist, umfassend das Bereitstellen einer Körperflüssigkeitsprobe aus einem Individuum, von dem bekannt ist oder vermutet wird, dass es an einer Infektion leidet, und Erhalten eines Empfindlichkeit-Scores, der die Empfindlichkeit eines jeglichen Glukose-metabolisierenden Mikroorganismus, der in der Probe vorliegend ist, gegenüber dem antibiotischen Kandidatenmittel unter Verwendung eines Verfahrens nach einem der Ansprüche 1-12 wiederspiegelt, wobei der Empfindlichkeit-Score die Wirksamkeit des antibiotischen Kandidatenmittels bei der Behandlung des Gesundheitszustandes anzeigt, wie beispielsweise eines Gesundheitszustandes, der aus der Gruppe bestehend aus Septikämie, Lungenentzündung, Endokarditis, Peritonitis und Meningitis ausgewählt ist.

14. Verwendung eines flüssigen Kulturmediums umfassend Hirn-Herz-Infusion oder Mueller-Hinton-Brühe enthaltend Glukose in einer Konzentration von 1 mg Glucose pro mL Kulturmedium, bei einem Verfahren nach einem der Ansprüche 1-13.

## Revendications

1. Procédé d'analyse de la sensibilité d'un microorganisme métabolisant le glucose à au moins un agent antibiotique, comprenant
a) la culture dudit microorganisme métabolisant le glucose dans un milieu de culture de référence, comprenant du glucose et ne comprenant pas l'agent antibiotique ; et la mesure de la concentration de glucose dans le milieu de culture de référence pour obtenir des données de concentration de glucose résolu dans le temps, ladite culture s'étendant sur une période de temps suffisante pour observer une diminution de la concentration de glucose dans le milieu de culture de référence ;
b) la culture simultanée dudit microorganisme métabolisant le glucose dans un milieu de culture contenu dans un récipient de milieu de culture, en présence d'une concentration prédéterminée d'un agent antibiotique et en présence de glucose ;
c) la mesure de la concentration en glucose du milieu de culture dans ledit récipient de milieu de culture au moins lorsque la concentration en glucose dans le milieu de culture de référence est descendue en dessous d'un seuil prédéterminé ; et
d) le calcul d'un score de sensibilité reflétant la sensibilité du micro-organisme métabolisant le glucose à l'agent antibiotique sur la base d'une relation entre la concentration prédéterminée de l'agent à activité antibiotique, les données de concentration de glucose obtenues à l'étape c), et les données de concentration de glucose résolu dans le temps obtenues à l'étape a).

2. Procédé selon la revendication 1, dans lequel l'étape c) comprend l'obtention de données de concentration de glucose résolu dans le temps pour le milieu de culture dans le récipient de milieu de culture pendant au moins une partie de la période de temps pendant laquelle le microorganisme métabolisant le glucose est cultivé.

3. Procédé selon l'une quelconque des revendications 1 à 2, comprenant en outre l'obtention de données de référence de concentration de glucose résolu dans le temps pour le milieu de culture dans un récipient de référence ne comprenant aucun microorganisme métabolisant le glucose.

4. Procédé selon l'une quelconque des revendications 1 à 3, dans lequel ledit microorganisme métabolisant le glucose est cultivé dans une pluralité de récipients de milieu de culture, et dans lequel ledit agent antibiotique est présent dans chaque récipient de milieu de culture dans une quantité qui est prédéterminée pour chaque récipient de milieu de culture, éventuellement dans lequel la pluralité de récipients de milieu de culture contiennent ensemble une série de concentrations de l'agent antibiotique.

5. Procédé selon l'une quelconque des revendications 1 à 4, dans lequel ledit microorganisme métabolisant le glucose est cultivé dans une pluralité de récipients de milieu de culture, et dans lequel les récipients de milieu de culture contiennent ensemble une pluralité de différents agents antibiotiques.

6. Procédé selon l'une quelconque des revendications 1 à 5, dans lequel le score de sensibilité est un score de sensibilité qualitatif, tel qu'un score de sensibilité qualitatif défini pour une concentration spécifique d'un agent spécifique à activité antibiotique.

7. Procédé selon l'une quelconque des revendications 1 à 5, dans lequel le score de sensibilité est un score de sensibilité quantitatif, tel qu'une concentration minimale inhibitrice (MIC) ou une concentration minimale létale (MLC).

8. Procédé selon la revendication 7, dans lequel le score de sensibilité est calculé sur la base de données de concentration de glucose résolu dans le temps provenant d'une pluralité de récipients de milieu de culture comprenant le micro-organisme et comprenant ensemble une dilution en série de l'agent antibiotique, un récipient de milieu de culture de référence comprenant le microorganisme et ne comprenant pas l'agent antibiotique, et un récipient de milieu de culture à blanc ne comprenant pas le microorganisme et ne comprenant pas l'agent antibiotique.

9. Procédé selon l'une quelconque des revendications précédentes, dans lequel la mesure de la concentration en glucose implique une mesure de fréquence indicative de la concentration en glucose, éventuellement dans lequel la mesure de fréquence est une mesure d'une fréquence de résonance du récipient de milieu de culture, et / ou dans lequel la mesure de fréquence est une mesure de haute fréquence électrique.

10. Procédé selon l'une quelconque des revendications 1 à 8, dans lequel la mesure de la concentration en glucose est effectuée avec une électrode à enzyme.

11. Procédé selon l'une quelconque des revendications 1 à 10, dans lequel la concentration de glucose est mesurée dans le récipient de milieu de culture.

12. Procédé selon l'une quelconque des revendications 1 à 11, dans lequel le milieu de culture et le milieu de culture de référence sont le bouillon Mueller-Hinton ou l'infusion cœur-cervelle, supplémenté en glucose.

13. Procédé d'évaluation de l'efficacité d'un agent antibiotique candidat dans le traitement d'un condition médicale provoquée ou compliquée par une infection, comprenant la fourniture d'un échantillon de fluide corporel d'un sujet connu ou suspecté d'être atteint d'une infection et l'obtention d'un score de sensibilité reflétant la sensibilité de tout microorganisme métabolisant le glucose présent dans ledit échantillon audit agent antibiotique candidat à l'aide d'un procédé selon l'une quelconque des revendications 1 à 12, dans lequel le score de sensibilité indique l'efficacité de l'agent antibiotique candidat dans le traitement de l'état médical, tel qu'un état médical choisi dans le groupe constitué par la septicémie, la pneumonie, l'endocardite, la péritonite et la méningite.

14. Utilisation d'un milieu de culture liquide comprenant une infusion cœur-cervelle ou un bouillon Mueller-Hinton comprenant du glucose à une concentration de 1 mg de glucose par ml de milieu de culture, dans un procédé selon l'une quelconque des revendications 1 à 13.
